# EUROPÄISCHE PATENTSCHRIFT

(11) **EP 2 330 978 B1**
(45) Veröffentlichungstag und Bekanntmachung des Hinweises auf die Patenterteilung: **13.11.2013**
(21) Anmeldenummer: 09777188.5
(22) Anmeldetag: 14.07.2009
(51) Int. Cl.: A61B 6/04, G21F 3/02, A61L 15/54, D04B 1/24

(54) **LINER ZUM UMGEBEN EINES KÖRPERTEILS WÄHREND EINES BILDGEBUNGSVERFAHRENS**
LINER FOR ENCLOSING A BODY PART DURING AN IMAGING PROCEDURE
MANCHON DESTINE A ENTOURER UNE PARTIE CORPORELLE PENDANT UN PROCEDE D'IMAGERIE

(30) Priorität: 14.07.2008 DE 102008032992
(43) Veröffentlichungstag der Anmeldung: 15.06.2011
(73) Patentinhaber: Pro Thesis GmbH, 86154 Augsburg (DE)
(72) Erfinder: SCHOTTDORF, Bernd, 89561 Dischingen (DE)
(74) Vertreter: Stammberger, Tobias
(86) Internationale Anmeldenummer: PCT/EP2009/005119
(87) Internationale Veröffentlichungsnummer: WO 2010/006769

(56) Entgegenhaltungen:
- US-A- 3 310 053
- US-A- 5 329 932
- US-A1- 2006 108 548

## Beschreibung

### GEBIET DER ERFINDUNG

Die vorliegende Erfindung betrifft einen Liner zum wenigstens teilweisen Umgeben eines Körperteils während eines Bildgebungsverfahrens, die Verwendung des Liners zum Simulieren der Gewichtskraft des Körperteils während eines Bildgebungsverfahrens und die Verwendung des Liners zur Erstellung eines virtuellen 3D-Modells eines Körperteils.

### HINTERGRUND DER ERFINDUNG

Bildgebungsverfahren haben eine breite Anwendung in der medizinischen Diagnostik gefunden. So lassen sich damit Informationen über die Geometrie eines Körperteils oder die Gewebeverteilungen im Inneren eines Organismus darstellen und ermitteln. Zudem kann erkranktes Gewebe, wie beispielsweise Tumore oder Entzündungsherde, identifiziert werden. Besonders häufig eingesetzte Bildgebungsverfahren sind die Magnetresonanztomographie (MRT) und die Computertomographie (CT). Bei der MRT basiert die Entstehung eines Bildes auf der für jedes Gewebe charakteristischen Wechselwirkung des Gewebes mit einer angelegten elektromagnetischen Strahlung. Bei der CT basiert der Kontrast zwischen verschiedenen Geweben auf der für jedes Gewebe charakteristischen Abschwächung der Röntgenstrahlung.

Konstruktionsbedingt befindet sich bei der Bildaufnahme der Patient oft in liegender Position. Durch das Einwirken der Schwerkraft werden verschiedene Gewebe verschoben, so dass sich eine Querformung des abzubildenden Körperteils ergibt. Bei einer anschließenden Auswertung der Bildinformation über die Lage der Gewebe kann diese Querformung zu Fehleinschätzungen führen. Insbesondere bei Untersuchungen, die Aufschluss über die Geometrie und Lage oder die Verteilung der Gewebearten geben sollen kann diese Querformung zu einer falschen Schlußfolgerung führen.

Bekannt sind Vorrichtungen, die den Körper oder den Körperteil während des Bildgebungsverfahrens stützen oder fixieren. Dabei wird der Körper oder Körperteil derart positioniert, dass die gewünschten Aufnahmen erhalten werden können oder der Aufnahmeprozess für den Patienten so bequem wie möglich gestaltet werden kann. Die Fixierung des Körpers oder Körperteils dient auch einer Reduktion von Bewegungsartefakten. Diese bekannten Vorrichtungen können eine schwerkraftbedingte Querformung der abzubildenden Körperteile nicht verhindern.

Zudem sind Liner bekannt, die zur Verbesserung des Tragekomforts von Prothesenpatienten zwischen Stumpf und Prothesenschaft getragen werden. Hierbei bietet das Liner-Material mechanischen Schutz vor Reibung für die Stumpfhaut und zusätzlich eine Oberfläche, die am Schaft besser anhaftet als die Haut und dadurch eine bessere Kraftübertragung ermöglicht. Typischerweise für die Herstellung der Liner verwendete Materialien, wie Polyurethan, Silikon oder Textilfasermaterialien lassen sich schlecht in Bildgebungsverfahren darstellen.

Aufgrund der physikalischen Prinzipien, auf denen die Bildgebungsverfahren basieren, ist das Abbildungsvermögen jedes Bildgebungsverfahrens für bestimmte Anwendungen begrenzt. Beispielsweise können benachbart angeordnete Gewebe mit gleichen oder ähnlichen physikalischen Eigenschaften in den Bildaufnahmen nur schwer unterschieden werden. In diesem Fall ist eine Segmentierung der Bilddaten, also die Zuordnung von Bildpixeln zu den einzelnen Geweben, schwierig und zeitintensiv.

US 2006/0108548 offenbart ein Strahlungsabschwächungssystem zum Umgeben eines Körperteils während eines Bildgebungsverfahrens, wobei das Strahlungsabschwächungssystem geeignet ist, den Körperteil im Wesentlichen in einer bestimmten Form zu halten und wobei das Strahlungsabschwächungssystem an der Hautoberfläche des Körperteiles annähernd formschlüssig anlegbar ist, wobei das Strahlungsabschwächungssystem ein Material aufweist, das ein Grundmaterial und ein Kontrastmittel umfasst.

Der Kontrast zwischen benachbarten Geweben kann zwar durch Verabreichung eines gewebespezifisch wirkenden Kontrastmittels erhöht werden. Dieses Vorgehen eignet sich jedoch nicht, um benachbarte gleichartige Gewebe verschiedener Körperteile, insbesondere Hautschichten, voneinander zu trennen.

Liegen zwei Körperteile mit derselben Gewebeart direkt nebeneinander, so kann die Grenzfläche zwischen beiden Körperteilen anhand der Bildaufnahmen nur schwer identifiziert werden.

Der Erfindung liegt daher die Aufgabe zugrunde, einen Liner für Bildgebungsverfahren bereit zu stellen, welcher die beschriebenen Nachteile überwindet.

### ZUSAMMENFASSUNG DER ERFINDUNG

Erfindungsgemäß wird diese Aufgabe durch den Gegenstand der unabhängigen Ansprüche gelöst. Varianten und bevorzugte Ausführungsformen ergeben sich aus den abhängigen, der nachfolgenden Beschreibung und den Zeichnungen.

Ein Aspekt der Erfindung betrifft einen Liner zum wenigstens teilweisen Umgeben eines Körperteils während eines Bildgebungsverfahrens. Der Liner ist geeignet, den Körperteil im Wesentlichen in einer bestimmten Form zu halten. Gleichzeitig ist der Liner an der Hautoberfläche des Körperteils annähernd formschlüssig anlegbar. Zudem weist der Liner ein Material auf, das ein Grundmaterial umfasst, in dem wenigstens ein Kontrastmittel im Wesentlichen homogen verteilt ist, das geeignet ist, bei Einsatz des Liners am Körperteil in dem Bildgebungsverfahren den Kontrast des Linermaterials bezüglich Hautgewebe in den generierten Bilddaten relativ zum Kontrast des Grundmaterials bezüglich Hautgewebe zu verstärken.

Vorteilhafterweise werden dreidimensionale Bilddaten in dem Bildgebungsverfahren mittels einer Magnetresonanztomographie (MRT) und/oder einer Computertomographie (CT) aufgenommen. Aber auch andere Bildgebungsverfahren, wie beispielsweise die Positronen-Emissions-Tomographie (PET) oder Ultraschall-Verfahren können zur Anwendung kommen.

Der abzubildende Körperteil umfasst im Allgemeinen verschiedene Gewebearten, wie Haut, Fett, Muskel und Knochen und ist durch eine äußere Hautschicht begrenzt.

Der Liner besteht bevorzugt aus einem Material, welches elastisch, also reversibel verformbar ist, sodass sich der Liner formschlüssig, also insbesondere ohne Lufteinschlüsse zu bilden, an einem Körperteil eines Patienten anbringen lässt. Dies bedeutet, dass Unebenheiten der Hautoberfläche detailgetreu durch den Liner nachempfunden werden. Insbesondere bei Narbengewebe kann sich die hohe Anschmiegbarkeit und Dehnbarkeit des Linermaterials vorteilhaft auswirken. Zusätzlich weist das Linermaterial auch eine bestimmte Zug- und Druckfestigkeit auf, um das abzubildende Körperteil zu formen. Dabei übt der Liner bevorzugt einen gleichmäßigen Druck auf den Körperteil aus. Die Druckverteilung kann alternativ auch inhomogen über die Oberfläche des Körperteils verteilt sein.

Bevorzugt handelt es sich bei dem Grundmaterial um eine Polymer-Verbindung. Neben dem Grundmaterial, welches für die elastischen Eigenschaften des Liners verantwortlich ist, umfasst das Linermaterial ein Kontrastmittel. Bevorzugt handelt es sich hierbei um eine Substanz, die in einem Bildgebungsverfahren ein Signal erzeugt, welches sich deutlich von dem Signal des Hautgewebes unterscheidet. Dabei kann das Signal im Vergleich zum Signal des Hautgewebes stärker oder schwächer sein. Bevorzugt wird die Signalstärke des Linermaterials durch das Kontrastmittel auf einen Wert zwischen den Signalstärken von Knochen- und Muskelgewebe angehoben, um eine anschließende automatische Segmentierung zu ermöglichen. Bevorzugt werden beispielsweise für die Magnetresonanztomographie Verbindungen eingesetzt, die Gadolinium oder superparamagnetisches Eisenoxid enthalten. Aber auch Mangan- oder Nickelverbindungen können eingesetzt werden. Für die Computertomographie werden vorteilhafterweise jodhaltige Verbindungen eingesetzt.

Durch die derart zwischen Haut und Liner erzeugte Kontrastverstärkung wird in den aufgenommenen Darstellungen die Identifizierung der Außenfläche des Körperteils vorteilhaft vereinfacht. Beispielsweise können zwei Körperteile bei einer Bildaufnahme direkt neben einander liegen. Ohne Einsatz des erfindungsgemäßen Liners grenzt das Hautgewebe des ersten Körperteils unmittelbar an das Hautgewebe des zweiten Körperteils. Dies ist beispielsweise bei Aufnahmen eines Oberschenkels eines männlichen Patienten und seiner Geschlechtsorgane der Fall. In den Bildaufnahmen werden die Hautgewebe der Körperteile mit demselben oder einem ähnlichen Grauwert dargestellt, da beide Hautgewebe annähernd dieselben Signale erzeugen. Eine Trennung der Körperteile in den Bildern durch Bestimmen der Oberflächen kann daher nur schwer erfolgen. Zudem erschwert das Bildrauschen ein Erkennen von minimalen Helligkeitsunterschieden in den Darstellungen. Durch die Kontrastverstärkung zwischen Linermaterial und dem Hautgewebe des umgebenen Körperteils ist die Oberfläche des umgebenen Körperteils in den 3D-Bilddaten identifizierbar. Die Identifizierung der Körperteiloberfläche kann manuell, semiautomatisch oder automatisch erfolgen. Bei automatischen Segmentierungs-Algorithmen werden einzelne Pixel in den Darstellungen den verschiedenen Gewebearten zugeordnet. Die Zuordnung kann beispielsweise anhand einer Schwellwert-Analyse der Grauwerte für die einzelnen Pixel erfolgen. Insbesondere kann sich die Kontrastverstärkung zwischen dem Linermaterial und dem Hautgewebe positiv bei der Anwendung von automatischen Segmentierungs-Algorithmen zur Identifizierung einer Körperteiloberfläche auswirken. Durch die Kontrastverstärkung wird dafür insbesondere der Einfluss des Bildrauschens reduziert. Je deutlicher der Kontrast zwischen Linermaterial und dem vom Liner umgebenen Gewebe ausfällt, umso besser können automatische Segmentierungs-Algorithmen zum Einsatz kommen. Durch eine deutliche Abgrenzung des Hautgewebes von umliegenden anderen Körperteilen kann anhand der segmentierten Körperteiloberfläche insbesondere ein genaues virtuelles 3D-Modell des Körperteils erstellt werden, das die Oberflächenform des Körperteils detailgetreu beschreibt.

Gemäß einer Ausführungsform der Erfindung ist die bestimmte Form, in der der Körperteil gehalten wird, die Form des Körperteils, die sich einstellt, wenn sich der Körperteil in einer definierten Lage oder Haltung befindet. Insbesondere wird der Körperteil in der Form gehalten, die der Körperteil annimmt, wenn sich der Körper, in stehender, also aufrechter Position befindet. Insbesondere ist die Darstellung von Beinen bevorzugt in aufrechter Haltung interessant, in welcher beispielsweise beim Gehen große Kräfte wirken. Bei Bildgebungsverfahren wie der CT oder der MRT wird der Patient dagegen bei der Bildaufnahme typischerweise liegend in der Aufnahmevorrichtung gelagert, was zur Verformung des abzubildenden Körperteils führen kann. Mit dieser Ausführungsform wird damit der Körperteil in der für die weitere Verarbeitung der Bilddaten relevanten Haltung abgebildet. Eine solche bevorzugte Weiterverarbeitung ist beispielsweise die Erstellung eines virtuellen 3D-Stumpfmodells, anhand dessen ein Prothesenschaft erstellt wird.

Gemäß einer weiteren Ausführungsform der Erfindung handelt es sich bei dem Körperteil um einen Extremitäten-Stumpf. Beispielswesie ist der Extremitäten-Stumpf ein Oberschenkel-Stumpf. Zudem ist der Liner geeignet, den Stumpf vollständig zu umgeben.

Bei der Herstellung von Prothesenschäften für einen Amputationspatienten ist es besonders vorteilhaft, detaillierte und der Realität entsprechende virtuelle Darstellungen des Stumpfes und daraus insbesondere 3D-Modelle des Stumpfes zu erzeugen, anhand derer eine passgenaue Form eines Prothesen-Schaftes konstruiert werden kann. Dafür kann der Verlauf der Hautoberfläche mittels des Liners in den 3D-Bilddaten exakt in den Aufnahmen des Bildgebungsverfahrens dargestellt und anhand einer Segmentierung rekonstruiert werden. Insbesondere bei männlichen Transfemoralamputierten kann anhand des Liners medial eine Separierung des Stumpf-Gewebes von den Geschlechtsorganen automatisch erfolgen.

Bevorzugt einspricht die bestimmte Form, in welcher der Stumpf gehalten wird, der Form des Stumpfes, in welcher sich der Stumpf in aufrechter, also vertikaler Haltung befindet. Dies ist insbesondere die Position eines Stumpfes, in welcher die größten mechanischen Kräfte auf den Stumpf einwirken. Insbesondere ein Oberschenkel-Stumpf wird beim Gehen mit Hilfe einer Prothese starken Belastungen ausgesetzt.

Durch die charakteristischen Formeigenschaften des Linermaterials kann der Stumpf bei der Bildaufnahme in der bestimmten Form gehalten werden. Insbesondere verhindert der Liner im Wesentlichen eine Querformung des Körperteils in einer horizontalen Lage bzw. liegenden Position des Stumpfes. Dies ist bei Bildgebungsverfahren, bei denen der Stumpf liegend abgebildet wird, besonders von Vorteil. Die Querformung und Abflachung in liegender Position resultieren maßgeblich daraus, dass die Gewichtskraft senkrecht zur Körperachse an den Stumpf angreift. Mit am Körperteil angebrachtem Liner wird bevorzugt die Lage der verschiedenen Gewebe des Stumpfes in aufrechter Position beibehalten. Vorteilhafterweise wird dadurch eine verbesserte Darstellung der tatsächlichen Gewebeverteilungen des Stumpfes bei der Bildaufnahme erzielt. Dies ermöglicht eine bessere Diagnose und eine genauere Vermessung des Stumpfes, beispielsweise mittels einer dafür vorgesehenen Software.

Gemäß einer weiteren Ausführungsform der Erfindung umfasst das Grundmaterial des Liners Polyurethan, Silikon und/oder thermoplastische Elastomere oder Ähnliches. Jede als Grundmaterial verwendete Substanz weist charakteristische Materialeigenschaften auf. Polyurethan ist beispielsweise eine Polymerverbindung aus Polyolen und Polyisocyanaten. Sie zeichnet sich durch eine vergleichsweise hohe Viskoelastizität aus und eignet sich daher bevorzugt für eine exakte Nachbildung einer ungleichmäßigen, insbesondere narbigen Hautoberfläche. Die mechanischen Eigenschaften können vorteilhafterweise durch eine Variation der verwendeten Polyol-Verbindung und/oder der Konzentrationen der beiden Ausgangsstoffe an die spezifischen Anforderungen einer Untersuchung mittels bildgebendem Verfahren oder der Anatomie des Körperteils angepasst werden. In polymerisierter Form, in der die Einzelkomponenten über Urethangruppen dreidimensional vernetzt sind, weisen Polyurethane zudem keine toxischen Eigenschaften auf, sodass das Material in der Medizin und/oder der Medizintechnik anwendbar ist. Polyurethan kann beispielsweise als Gel, Elastomer oder Polyurethan-Schaum für die Herstellung eines Liners verwendet werden.

Silikone hingegen weisen typischerweise eine hohe Zugfestigkeit auf, welche sich vorteilhaft auswirkt, wenn bei einem Körperteil in liegender Position mit einer besonders hohen Querverformung zu rechnen ist. Dies ist insbesondere bei adipösen Patienten der Fall. Auch Silikon ist ungiftig und ruft nur selten allergische Reaktionen hervor.

Bevorzugt können auch thermoplastische Elastomere als Grundmaterial für den Liner verwendet werden, wenn dieser nachträglich weiter an den Körperteil angepasst werden muss, denn durch Zufuhr von Wärme kann der Liner nachträglich verformt werden.

Ein weiteres Ausführungsbeispiel betrifft einen Liner, der bevorzugt aus einer Schicht des Grundmaterials geformt ist, in welcher homogen verteilt Kontrastmittel-Partikel oder Kontrastmittelmoleküle eingebracht sind. Das Kontrastmittel liegt bevorzugt in einer Form bzw. Verbindung vor, welche mit dem Grundmaterial im noch unpolymerisierten Zustand homogen mischbar ist. Vorteilhafterweise stört und/oder inhibiert das beigefügte Kontrastmittel die Polymerisierungsreaktion des Grundmaterials nicht. Beispielsweise eigenen sich insbesondere Kontrastmittel in ethanolischen Lösungsmitteln oder Kontrastmittel in aromatischen Verbindungen nicht für die Herstellung eines Polyurethan-Liners. Beim Herstellungsprozess eines Liners können bevorzugt pulverförmige Kontrastmittelpartikel in das Grundmaterial eingebracht werden, welches anschließend polymerisiert wird und dadurch die Kontrastmittelpartikel einschließt. Das Kontrastmittel kann alternativ auch flüssig sein. Beispielsweise kann eine Emulsion aus unpolymerisiertem Grundmaterial und Kontrastmittellösung hergestellt werden. Bevorzugt wird das Kontrastmittel in den Zwischenräumen des Polymer-Netzes angelagert, sodass die sterische Ordnung der Polymere nicht beeinflusst wird. Alternativ kann die Kontrastmittelverbindung an der chemischen Polymerisationsreaktion beteiligt sein und eine chemische Bindung mit dem Grundmaterial eingehen.

Gemäß einer weiteren Ausführungsform der Erfindung ist das Kontrastmittel ein Kontrastmittel, das in zwei oder mehr Bildgebungsverfahren kontrastverstärkend wirkt. Bevorzugt handelt es sich bei den wenigstens zwei Bildgebungsverfahren um die Magnetresonanztomographie und die Computertomographie. Beispielsweise kann dafür ein Kontrastmittel verwendet werden, welches Wassermoleküle enthält.

Bevorzugt handelt es sich bei den Kontrastmitteln um nicht toxische Substanzen und/oder um nicht über die Haut resorbierbare Substanzen, um eine gute Materialverträglichkeit des Liners zu erreichen. Um die Sicherheit zu erhöhen, kann zusätzlich die Innenfläche sowie die Außenfläche des Liners vorteilhafterweise mit einer Deckschicht des Grundmaterials ohne Kontrastmittel überzogen sein, sodass ein Kontakt eines Orthopädietechnikers und/oder des Patienten mit dem Kontrastmittel ausgeschlossen werden kann. Alternativ kann die Deckschicht aus Textilfasern oder Ähnlichem bestehen.

Gemäß einer weiteren Ausführungsform der Erfindung umfasst das Kontrastmittel Jod. Bevorzugt handelt es sich bei der jodhaltigen Verbindung um Kalium-Jodid. Dieses liegt als Feststoff vor und kann dem Grundmaterial beigefügt werden. Das Jod bewirkt eine verstärkte Absorption der Röntgenstrahlung, was bei der CT in einer Kontrastverstärkung zwischen Liner und umliegendem Hautgewebe resultiert.

Gemäß einer weiteren Ausführungsform der Erfindung beträgt das Massenverhältnis des Jods zum Grundmaterial mindestens 1 x 10⁻⁵. Vorteilhafterweise wird dem Grundmaterial eine Mindestkonzentration des Kontrastmittels beigefügt, um eine merkliche Kontrastverstärkung zu erzielen. Die Konzentration des Kontrastmittels wird derart erhöht, sodass anhand der bewirkten Kontrastverstärkung mittels der erzeugten dreidimensionalen Bildinformationen eine automatische Segmentierung der verschiedenen Schichten in den Bildaufnahmen durch einen SegmentierungsAlgorithmus erfolgen kann. Um die Segmentierung zu optimieren, wird die Konzentration des Kontrastmittels vorteilhafterweise so gewählt, dass sich die daraus resultierende Signalstärke des Linermaterials von den zu segmentierenden Gewebearten, wie Haut, Muskel oder Knochen unterscheidet, um eine eindeutige Zuordnung der Pixel zum Liner und/oder einer Gewebeart zu ermöglichen. Das Linermaterial liefert bevorzugt eine Signalstärke, welche zwischen der Signalstärke von Muskelgewebe und der Signalstärke von Knochengewebe bei CT-Messungen liegt.

Gemäß einer weiteren Ausführungsform der Erfindung umfasst das Kontrastmittel Glyceroltrinitrat. Gemäß einer besonderen Ausführungsform der Erfindung beträgt das Massenverhältnis des Glyceroltrinitrats zum Grundmaterial mindestens 4 x 10⁻⁵, bei welcher sich die zu erzielende Kontrastverstärkung zwischen Hautschicht und Liner in den MRT-Schnittbildern bemerkbar zeigt. Vorteilhafterweise wird die Konzentration des Glyceroltrinitrats auf einen optimalen Wert erhöht, sodass ein automatischer Segmentierungs-Prozess durchgeführt werden kann, welcher die durch die Hautschicht gebildete Außenfläche des Körperteils ermittelt. Auch bei MRT-Aufnahmen wird die Signalstärke des Linermaterials durch das Kontrastmittel bevorzugt auf einen Wert zwischen den Signalstärken von Knochen- und Muskelgewebe angehoben. Jedenfalls unterscheidet sich die Signalstärke derart von der Signalstärke anderer, zu segmentierender Gewebearten, um bei der Segmentierung eine eindeutige Zuordnung der Pixel zum Linermaterial zu ermöglichen.

Gemäß einer weiteren Ausführungsform der Erfindung ist in dem Grundmaterial des Liners ein weiteres Kontrastmittel für je ein weiteres Bildgebungsverfahren homogen verteilt, wobei sich die enthaltenen Kontrastmittel in ihrer kontrastverstärkenden Wirkung im Wesentlichen nicht beeinträchtigen. Insbesondere unter wirtschaftlichen Gesichtspunkten kann es vorteilhaft sein, einen Liner universell in mehreren verschiedenen Bildgebungsverfahren anwenden zu können. Bei einem individuell für einen Patienten angefertigten Liner ist es aus Kostengründen sinnvoll, dass Grundmaterial mit verschiedenen Kontrastmitteln herzustellen, sodass derselbe Liner in verschiedenen Untersuchungen ggf. zur Beantwortung unterschiedlicher Fragestellungen verwendet werden kann. Die aufwändige Fertigung eines neuen Liners für eine andere Aufnahmetechnik entfällt damit. Zudem braucht bei der Kombination mehrerer Kontrastmittel in einem Liner im Hinblick auf eine Produktion derartiger Liner lediglich ein Fertigungsprozess entwickelt zu werden. Dies ist zeit- und kostensparend.

Gemäß einer weiteren Ausführungsform der Erfindung ist der Liner als einseitig oder zweiseitig offener Strumpf ausgebildet. In der lediglich einseitig offenen Strumpfform ist der Liner bevorzugt strumpfartig an einem Extremitäten-Stumpf, insbesondere einen Oberschenkel-Stumpf anbringbar. Diese Ausführungsform eignet sich alternativ zur Verwendung an Oberarm-, Unterarm- und Unterschenkelstümpfen.

Die Form des strumpfartigen Liners ist an die jeweiligen physiologischen Gegebenheiten eines Stumpfes beliebig anpassbar, indem eine andere Matrix für das Herstellungsverfahren mit einer Press-, Guss- oder Spritzgusstechnik verwendet wird. Die Matrixformen können vorteilhafterweise Standardformen sein, die bei einer Vielzahl von Patienten angewendet werden können. Bevorzugt kann eine Linerform auch individuell für einen Patienten hergestellt werden, insbesondere dann, wenn eine Nachbildung der Stumpfform mittels einer Standardmatrix nicht erreicht werden kann. Durch die einseitig geschlossene Form des Liners kann die gesamte Oberfläche des Stumpfes, insbesondere der Verlauf des Narbengewebes an der Amputationsstelle abgebildet werden. Dies ist besonders vorteilhaft, wenn es darum geht, die Oberflächenform des Stumpfes genau zu ermitteln, um sensible und belastbare Areale der Oberfläche zu identifizieren. Die Ergebnisse dieser Untersuchungen können bevorzugt bei der Herstellung eines Prothesen-Schaftes heran gezogen werden.

Eine alternative Ausführungsform ist die zweiseitig offene Strumpfform des Liners. Diese Linerform kann vorteilhafterweise an eine beliebige Position einer Extremität und/oder eines Extremitäten-Stumpfes angebracht werden. Dies ist insbesondere dann vorteilhaft, wenn eine Extremität in einem körpernahen Bereich abgebildet werden soll, derart, dass sie von umliegenden Geweben des Körpers separierbar ist. Zudem können auf diese Art verschiedene Standardformen bei einer Vielzahl von Patienten eingesetzt werden. Durch die Wahl einer Standardform des Liners mit einem spezifischen Grundmaterial kann der Orthopädietechniker vorgeben, wie gut die Oberfläche einer Extremität abgebildet werden soll und/oder wie gut die Extremität in seiner der aufrechten Position entsprechenden Form gehalten werden soll.

Eine weitere Ausführungsform betrifft einen Liner in Streifenform, welche ähnlich einer Bandage bzw. Binde um einen Körperteil gewickelt werden kann. Dies ermöglicht es dem Orthopädietechniker, den Druck und/oder die Zugspannung, welche der Liner auf den Körperteil ausübt, individuell einzustellen, indem er den Liner fester oder lockerer um den Körperteil wickelt.

Gemäß einer weiteren Ausführungsform der Erfindung beträgt die Dicke des Linermaterials mindestens 5 mm. Um die auf Wechselwirkungen der Makromoleküle auf mikroskopischer Ebene beruhenden makroskopischen mechanischen Eigenschaften des Grundmaterials, wie beispielsweise die Zugfestigkeit, die Dehnbarkeit und/oder die Härte optimal zu entfalten, muß das Linermaterial eine entsprechende Mindestdicke aufweisen. Eine Mindestdicke von etwa 5 mm hat sich dabei aufgrund der typischerweise verwendeten Bildauflösung als besonders vorteilhaft bei der Anwendung bei einem Bildgebungsverfahren erwiesen.

Ein weiterer Aspekt der Erfindung betrifft die Verwendung eines erfindungsgemäßen Liners in einer Zugvorrichtung zum Simulieren der an einem Körperteil unter verschiedenen Flexionswinkeln im Wesentlichen parallel zur Längsachse des Körperteils wirkenden Gewichtskraft des Körperteils während eines Bildgebungsverfahrens. Die Zugvorrichtung umfasst eine höhenverstellbar anordbare Umlenkrolle, über die mittels eines Gewichtes und eines Zugseils eine in Richtung und Stärke vordefinierbare Zugkraft auf den mit dem Zugseil verbundenen Liner ausübbar ist, sodass dadurch verschiedene Flexionswinkel des vom Liner umgebenen Körperteils relativ zum Körper einstellbar sind. Bei Anwendung der Zugvorrichtung mit dem Liner kann vorteilhaft eine Kraft auf einen Körperteil, insbesondere einen Stumpf ausgeübt werden. Diese Kraft entspricht im Wesentlichen der Gewichtskraft des Stumpfes, die auf den Stumpf wirken würde, wenn sich dieser in aufrechter Haltung befände.

Das Zugseil ist vorteilhafterweise aus einen uneleastischen Material, wie beispielsweise einer Textilfaser oder Leder gefertigt. Das Zugseil kann an beiden Enden über Ösen oder Haken mit dem Gewicht und/oder dem Liner verbindbar sein, auch jede beliebige andere Verbindung ist denkbar.

Das Zugseil bewirkt eine Zugkraft auf den Liner, welche durch die formschlüssige Verbindung mit dem ummantelten Stumpf denselben in liegender Position während eines Bildgebungsverfahrens idealerweise derart verformt, als wäre der Stumpf aufgerichtet. Die sich so ergebende Form und Lage der Gewebe im Stumpf wird durch das Bildgebungsverfahren abgebildet und kann vorteilhafterweise bei der Erstellung eines passgenauen Prothesenschaftes verwendet werden. Bevorzugt kann mittels der beschriebenen Zugvorrichtung auch die Flexion, also die Neigung des Stumpfes gegenüber dem Aufnahmetisch, auf welchem der Patient liegt, bei der Bildaufnahme eingestellt werden. Die Neigung des Stumpfes erfolgt ausgehend vom Hüftgelenk des Patienten. Dazu wird die Höhe der Umlenkrolle angepasst. Die Umlenkrolle kann dazu an einem Standfuß oder hängend angeordnet sein. Dadurch wird die Längsachse des Körperteils relativ zur Bildgebungsvorrichtung geneigt. Vorteilhafterweise können auf diese Weise gewünschte Aufnahmen und/oder Schnittbilder des Körperteils erzielt werden.

Ein weiterer Aspekt der Erfindung betrifft die Verwendung eines erfindungsgemäßen Liners zur Erstellung eines virtuellen 3D-Modells eines Körperteils in einem Verfahren mit den folgenden Schritten formschlüssiges Anlegen des Liners am Körperteil, Akquirieren von dreidimensionalen Bilddaten des Körperteils mittels eines Bildgebungsverfahrens, Segmentieren der Hautoberfläche in den dreidimensionalen Bilddaten anhand des verstärkten Kontrastes des Linermaterials bezüglich des Hautgewebes des Körperteils, und Rekonstruieren eines 3D-Modells anhand der segmentierten 3D-Bilddaten, wobei das 3D-Modell die Oberflächenform des Körperteils beschreibt.

Das formschlüssige Anbringen des Liners erfolgt vorzugsweise, indem der Liner auf links gedreht wird, sodass die Innenwand nach außen zeigt. Danach wird die Innenwand des Liners bevorzugt mit einer ethanolischen Lösung benetzt oder eingesprüht. Dies verhindert bevorzugt ein Aneinanderhaften der Linerseiten. Bei einem einseitig geschlossenen, strumpfartigen Liner insbesondere für einen Stumpf, wird der Liner am distalen Ende des Stumpfes angesetzt und über den Stumpf nach oben übergestreift. Dabei ist insbesondere darauf zu achten, dass sich keine Lufteinschlüsse bilden.

Bei einem beidseitig geöffneten Liner wird dieser zunächst über das Körperteil an die gewünschte Position gestreift und dann abgerollt, sodass auch hier der Lufteinschluss zwischen Hautgewebe und Linerinnenseite verhindert wird.

Im Anschluss wird der Patient auf dem Aufnahmetisch positioniert und es werden Bildinformationen über den Stumpf aufgenommen. Bevorzugt handelt es sich bei dem Bildgebungsverfahren um eine MRT, CT, PET oder dergleichen.

Die bei Anliegen eines erfindungsgemäßen Liners erzeugten Aufnahmen des Stumpfes werden im nächsten Schritt des Verfahrens verwendet, um die Hautoberfläche des Stumpfs zu segmentieren. Die dreidimensionalen Bilddaten eignen sich aufgrund des verstärkten Kontrastes zwischen dem Hautgewebe und dem Liner bevorzugt für eine automatische Segmentierung. Bei der Segmentierung wird jedes Pixel einer Gewebeart, insbesondere dem Hautgewebe, zugeordnet. Der Segmentierungsalgorithmus kann beispielsweise eine Graustufenseparierung der Pixel oder eine ähnliche Schwellwertanalyse durchführen. Der Segmentierungsalgorithmus kann alternativ ein Kantenalgorithmus sein. Das Kontrastmittel und/oder die Konzentration des Kontrastmittels können vorteilhafterweise an den verwendeten Segmentierungsalgorithmus angepasst werden, um optimale Segmentierungsergebnisse zu erzielen. Bevorzugt kann vorgesehen sein, dass ein Benutzer manuell in den Segmentierungsablauf eingreift.

Anschließend wird anhand der segmentierten dreidimensionalen Bilddaten eine virtuelles 3D-Modell des Stumpfes erzeugt. Dies kann bevorzugt mittels eines Interpolationsverfahrens erfolgen. Das 3D-Modell beschreibt vorteilhafterweise genau die Oberflächenform des Stumpfes.

Gemäß einer weiteren Ausführungsform der Erfindung umfasst die Verwendung des Liners zur Erstellung eines 3D-Modells in einem Verfahren auch ein Segmentieren der im Körperteil enthaltenen Gewebearten. Zusätzlich beschreibt das 3D-Modell die Gewebeverteilung in dem Körperteil. Dazu werden beim Segmentieren bevorzugt auch die Gewebearten Fett, Muskel und Knochen segmentiert. Das die gesamte dreidimensionale Gewebeverteilung in dem Stumpf beschreibende 3D-Modell kann dann vorteilhafterweise bei der Erstellung eines Prothesen-Schaftes herangezogen gezogen werden. Bevorzugt kann das 3D-Modell dazu einer Modifizierung unterzogen werden, welche die optimale Passform eines Schaftes für den abgebildeten Stumpf generiert. Die Modifizierung kann vorteilhafterweise anhand von hinterlegten wissensbasierten Regelsätzen automatisch erfolgen.

### KURZBESCHREIBUNG DER ZEICHNUNGEN

Nachstehend werden weitere bevorzugte beispielhafte Ausführungsformen der Erfindung anhand schematischer Zeichnungen näher erläutert. Dabei zeigen:
- Fig. 1a: eine schematische Darstellung eines Liners nach einem Ausführungsbeispiel der Erfindung;
- Fig. 1b: eine schematische Darstellung eines Liners nach einem Ausführungsbeispiel der Erfindung;
- Fig. 2a: einen Querschnitt eines Körperteils nach dem Stand der Technik;
- Fig. 2b: einen Querschnitt eines Körperteils umgeben von einem Liner nach einem Ausführungsbeispiel der Erfindung;
- Fig. 3: einen Querschnitt eines Körperteils und umliegende Körperbereiche nach einem Ausführungsbeispiel der Erfindung; und
- Fig. 4: eine Vorrichtung zur Simulation einer Gewichtskraft nach einem Ausführungsbeispiel der Erfindung.

### BESCHREIBUNG BEVORZUGTER AUSFÜHRUNGSFORMEN

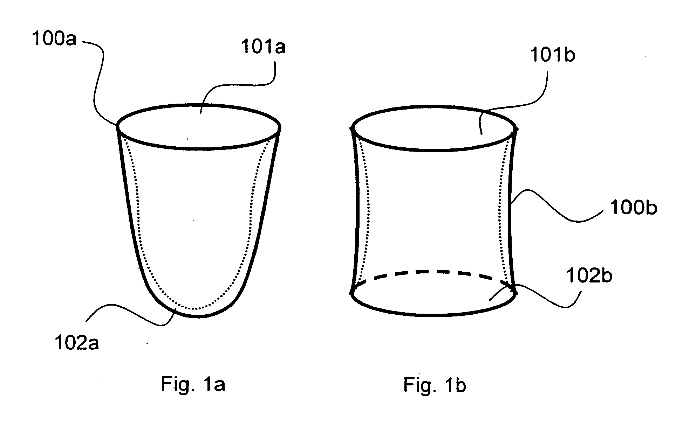

Fig. 1a zeigt schematisch und insbesondere nicht maßstabsgetreu einen Liner 100a. Der Liner 100a weist eine zylinderförmige Grundform auf. Die Länge des Liners sowie sein Durchmesser sind nicht festgelegt und den besonderen Anforderungen eines Patienten angepassbar. Insbesondere kann ein Liner 100a eine Maßanfertigung sein, welche beispielsweise Auswölbungen oder Verengungen der Mantelfläche in bestimmten Bereichen aufweist. Alternativ kann die Längsachse des Liners gekrümmt sein, um der Anatomie des Patienten gerecht zu werden. Der Liner verfügt im proximalen Bereich über eine Öffnung 101a, die dem Anziehen des Liners dient. Die gegenüberliegende Seite 102a ist gewölbt. Insbesondere kann der Liner zur Seite 102a hin konisch zulaufen. Die Dicke des Linermaterials ist im Wesentlichen konstant, reduziert sich jedoch zum offene Ende hin. Durch die einseitig geschlossene Form des Liners ist dieser bevorzugt bei der Abbildung von Extremitäten-Stümpfen zu verwenden. Insbesondere ergeben sich durch das Tragen des Liners bei der Bildaufnahme Vorteile bei der Abgrenzung eines Oberschenkels von den Geschlechtsorganen eines männlichen Patienten. Auch bei adipösen Patienten mit erhöhtem Körperumfang kann eine Trennung des Stumpfes von Gesäßbereichen oder Bauchgeweben durch den Liner bevorzugt erfolgen. Durch den gewölbten Abschluss des Liners im distalen Bereich kann so die Oberflächenform des Stumpfes vollständig nachgebildet und erfasst werden. Da auch das distale Ende des Stumpfes vom Linermaterial eingeschlossen wird, erfolgt eine gleichmäßig durch den Liner ausgeübte Druckverteilung auf den Stumpf. Der Umfang der Öffnung 101a kann ferner auch ellipsenförmig statt rund ausgestaltet sein, sodass sich auf verschiedenen Seiten des Liners verschiedene Höhen ergeben.

Fig. 1b zeigt schematisch und insbesondere nicht maßstabsgetreu einen Liner 100b. Dieser weist zwei Öffnungen 101b und 102b auf und ist daher insbesondere für die Abbildung einer vollständig intakten Extremität geeignet. Durch die Öffnung beider Seiten, ist es möglich, beim Anlegen den Liner über eine Hand oder einen Fuß zu führen. Die Position des Liners an der Extremität ist frei wählbar. So kann der Liner beispielsweise bis in den Schulterbereich eines Armes geführt werden und das Armgewebe von umliegenden Gewebearten des Brustkorbes trennen. Besonders bei adipösen Patienten mit vergleichsweise hohem Oberkörperumfang kann es vorteilhaft sein, Armgewebe durch den beschriebenen Liner vom Oberkörpergewebe abzugrenzen. Bei dem beschriebenen Liner können ferner die Umfänge der Deckflächen beliebige geschlossene Kurven beschreiben, sodass physiologische Besonderheiten eines Patienten berücksichtigt werden können. Der Liner kann ein beliebiges Verhältnis aus Länge und Durchmesser aufweisen. In besonderen Fällen kann die Mantelfläche speziell modifiziert werden. Insbesondere kann der Liner 100b längs konisch zulaufen und sich zur distalen Öffnung 102b hin verjüngen. Die Dicke des Linermaterials ist im Wesentlichen homogen und kann sich zu den geöffneten Deckflächen 101b, 102b hin verringern.

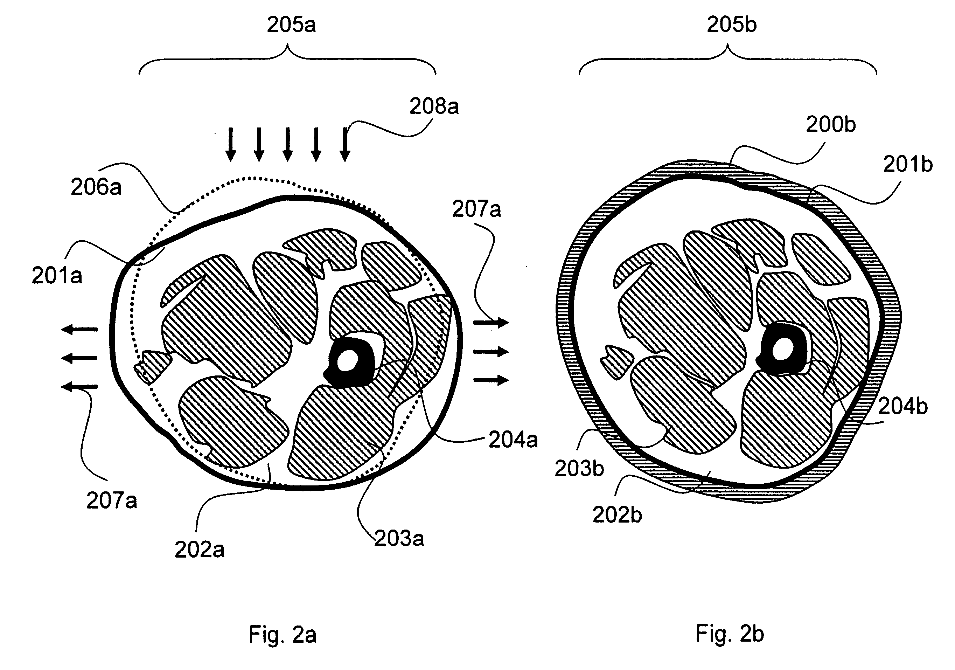

Fig. 2a zeigt einen Querschnitt einer Extremität 205a in horizontaler Lage bei Einwirkung der Schwerkraft. Dieser Querschnitt 205a kann beispielsweise ein Oberarm- oder Oberschenkelquerschnitt sein. Der Querschnitt 205a zeigt verschiedene Gewebearten, wie beispielsweise Haut 201a, Fett 202a, Muskel 203a und Knochen 204a. Bei einer horizontalen Lage einer Extremität, wie es bei medizinischen Bildgebungsverfahren üblich ist, ergibt sich durch die nun senkrecht zur Körperachse des Patienten einwirkende Gewichtskraft eine Querformung 207a und eine Abflachung 208a des Körperteils. Insbesondere entspricht die Lage der derart abgebildeten Gewebe nicht der natürlichen und die äußere Form des Körperteils nicht der natürlichen Form 206a, welche zur Verdeutlichung des Effektes gestrichelt dargestellt ist. Dieser Effekt kann eine anhand der Aufnahmen zu stellende Diagnose oder die Ergebnisse der geometrischen dreidimensionalen Vermessung einer Extremität maßgeblich verfälschen.

Fig. 2b zeigt einen Querschnitt einer Extremität 205b, in horizontaler Lage welcher von einem Liner 200b nach einem Ausführungsbeispiel der Erfindung umgeben ist. Dieser verhindert oder reduziert eine Abflachung und gleichzeitige Querformung der Extremität (siehe Fig. 2a). Die in der Extremität enthaltenen Gewebearten Haut 201b, Fett 202b, Muskel 203b und Knochen 204b befinden sich dadurch in ihrer natürlichen, einer aufrechten Haltung der Extremität entsprechenden, Lage. Auch die äußere Form entspricht im Wesentlichen dieser natürlichen Form in aufrechter Stellung des Körperteils. Dadurch können die Ergebnisse der Schaftvermessung anhand der aufgenommenen Bildinformationen exakter ausfallen, was sich beispielsweise bei der Herstellung eines Prothesenschaftes für einen Patienten vorteilhaft auswirkt.

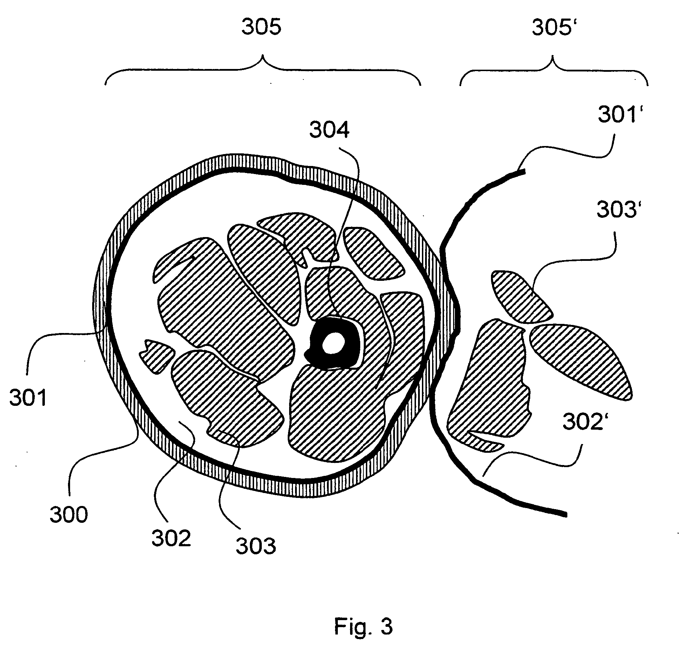

Fig. 3 zeigt einen Querschnitt einer Extremität 305, welcher von einem Liner 300 umgeben ist, der durch einen äußeren Druck die Extremität in einer bestimmten Form hält und im Wesentlichen die Lage der Gewebe 301 - 304 in der Extremität fixiert. Direkt neben der Extremität befindet sich ein umliegender Körperbereich 305', beispielsweise männliche Geschlechtsorgane oder Oberkörpergewebe, welcher ebenfalls verschiedene Gewebearten wie Haut 301', Muskel 303' und Fett 302' umfassen kann. Der Liner 300, welcher zwischen den Hautschichten der Extremität und des umliegenden Körperbereiches angeordnet ist, wird durch die kontrastverstärkende Wirkung des enthaltenen Kontrastmittels in den Bildaufnahmen deutlich abgebildet und ermöglicht es dadurch, die Hautschicht des umliegenden Körperbereiches von der Hautschicht der Extremität zu separieren. Die Hautoberfläche, welche die Außenfläche der Extremität bildet, kann somit mittels eines automatischen Segmentierungsalgorithmus bestimmt werden.

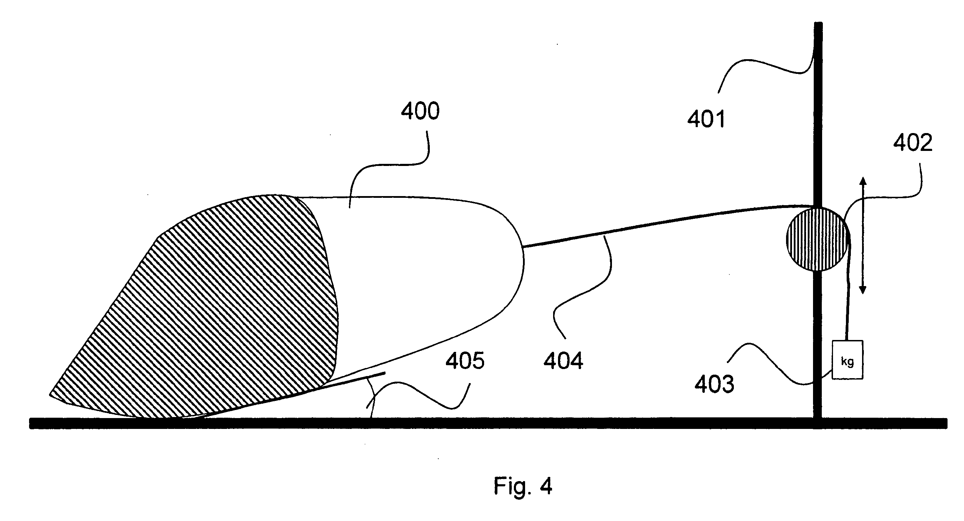

Fig. 4 zeigt eine Zugvorrichtung, welche während eines Bildgebungsverfahrens verwendet wird. Die Zugvorrichtung weist einen Standfuß 401, eine Rolle 402, ein Gewicht 403 und ein Seil 404 auf. Die Rolle 402 ist höhenverstellbar an dem Standfuß 401 befestigt. Über eine Höheneinstellung der Rolle kann der Neigungswinkel bzw. die Flexion 405 des abzubildenden Stumpfes in der Bildgebungseinrichtung eingestellt werden. Vorteilhafterweise liegt die Flexion eines Oberschenkelstumpfes beispielsweise in einem Bereich zwischen 5° und 20°. Der Neigungswinkel wird vom Orthopädietechniker anhand der anatomischen Gegebenheiten des Patienten vor der Bildaufnahme festgelegt. Über das Zugseil wird das Gewicht 403 mit dem Liner 400 verbunden. Bevorzugt handelt es sich bei dem Zugseil um nichtelastisches Material. Das Zugseil 401 wird über die Rolle 402 geführt und bewirkt, dass das Gewicht frei in der Luft hängt. So wird über das Zugseil eine Zugkraft auf den Liner ausgeübt, die im Wesentlichen längs der Stumpfachse wirkt. Die Masse des Gewichts entspricht vorteilhafterweise der Gewichtskraft, die auf den aufgerichteten Stumpf wirken würde. So können Bildaufnahmen des Stumpfes in einer Form erhalten werden, die im Wesentlichen der des aufrechten Stumpfes entspricht.

## Patentansprüche

1. Liner (100a; 100b; 200a; 200b; 300; 400) zum wenigstens teilweisen Umgeben eines Körperteils (205a; 205b; 305) während eines Bildgebungsverfahrens, der geeignet ist, die Identifizierung der Außenfläche des Körperteils in den generierten Bilddaten zu vereinfachen, wobei der Liner (100a; 100b; 200a; 200b; 300; 400) geeignet ist, den Körperteil (205a; 205b; 305) im Wesentlichen in einer bestimmten Form zu halten und wobei der Liner (100a; 100b; 200a; 200b; 300; 400) an der Hautoberfläche des Körperteils (205a; 205b; 305) annähernd formschlüssig anlegbar ist, wobei der Liner (100a; 100b; 200a; 200b; 300; 400) aus einem Material besteht, das ein Grundmaterial und wenigstens ein Kontrastmittel umfasst, wobei das Kontrastmittel im Wesentlichen homogen im Grundmaterial verteilt ist, und wobei das Kontrastmittel geeignet ist, bei Einsatz des Liners (100a; 100b; 200a; 200b; 300; 400) am Körperteil (205a; 205b; 305) in dem Bildgebungsverfahren den Kontrast des Linermaterials bezüglich Hautgewebe (201a; 201b; 301) in den generierten Bilddaten relativ zu einem Kontrast eines Linermaterials bestehend aus Grundmaterial bezüglich Hautgewebe (201a; 201b; 301) zu verstärken.

2. Liner (100a; 100b; 200a; 200b; 300; 400) gemäß Anspruch 1, wobei der Liner geeignet ist, den Körperteil (205a; 205b; 305) in der Form des Körperteils (205a; 205b; 305) zu halten, die sich einstellt, wenn sich der Körperteil (205a; 205b; 305) in einer definierten Lage oder Haltung, insbesondere bei stehendem Körper, befindet.

3. Liner (100a; 100b; 200a; 200b; 300; 400) gemäß Anspruch 1 oder 2, wobei der Liner geeignet ist, einen Extremitäten-Stumpf, insbesondere einen Oberschenkelstumpf, vollständig zu umgeben.

4. Liner (100a; 100b; 200a; 200b; 300; 400) gemäß einem der vorhergehenden Ansprüche, wobei das Grundmaterial Polyurethan, Silikon und/oder thermoplastische Elastomere umfasst.

5. Liner (100a; 100b; 200a; 200b; 300; 400) gemäß einem der vorhergehenden Ansprüche, wobei das Kontrastmittel ein Kontrastmittel ist, das in zwei oder mehr unterschiedlichen Bildgebungsverfahren kontrastverstärkend wirkt.

6. Liner (100a; 100b; 200a; 200b; 300; 400) gemäß einem der Ansprüche 1 bis 4, wobei das Kontrastmittel Jod umfasst.

7. Liner (100a; 100b; 200a; 200b; 300; 400) gemäß Anspruch 6, wobei das Massenverhältnis des Jods zum Grundmaterial mindestens 1 x 10⁻⁵ beträgt.

8. Liner (100a; 100b; 200a; 200b; 300; 400) gemäß einem der Ansprüche 1 bis 4, wobei das Kontrastmittel Glyceroltrinitrat umfasst.

9. Liner (100a; 100b; 200a; 200b; 300; 400) gemäß Anspruch 8, wobei das Massenverhältnis des Glyceroltrinitrats zum Grundmaterial mindestens 4 x 10⁻⁵ beträgt.

10. Liner (100a; 100b; 200a; 200b; 300; 400) gemäß einem der vorhergehenden Ansprüche, wobei in dem Grundmaterial des Liners (100a; 100b; 200a; 200b; 300; 400) wenigstens ein weiteres Kontrastmittel für je ein weiteres Bildgebungsverfahren homogen verteilt ist, wobei sich die Kontrastmittel in ihrer kontrastverstärkenden Wirkung im Wesentlichen nicht beeinträchtigen.

11. Liner (100a; 100b; 200a; 200b; 300; 400) gemäß einem der vorhergehenden Ansprüche, wobei der Liner (100a; 100b; 200a; 200b; 300; 400) als einseitig oder zweiseitig offener Strumpf ausgebildet ist.

12. Liner (100a; 100b; 200a; 200b; 300; 400) gemäß einem der vorhergehenden Ansprüche, wobei die Dicke des Linermaterials mindestens 5 mm beträgt.

13. Verwendung eines Liners (100a; 100b; 200a; 200b; 300; 400) gemäß einem der Ansprüche 1 bis 12 in einer Zugvorrichtung zum Simulieren der an einem Körperteil (205a; 205b; 305) unter verschiedenen Flexionswinkeln (405) im Wesentlichen parallel zur Längsachse des Körperteils (205a; 205b; 305) wirkenden Gewichtskraft des Körperteils (205a; 205b; 305) während eines Bildgebungsverfahrens, wobei die Zugvorrichtung eine höhenverstellbar anordbare Umlenkrolle (402) umfasst, über die mittels eines Gewichtes (403) und eines Zugseils (404) eine in Richtung und Stärke vordefinierbare Zugkraft auf den mit dem Zugseil (404) verbundenen Liner (100a; 100b; 200a; 200b; 300; 400) ausübbar ist, sodass dadurch verschiedene Flexionswinkel (405) des vom Liner (100a; 100b; 200a; 200b; 300; 400) umgebenen Körperteils (205a; 205b; 305) relativ zum Körper einstellbar sind.

14. Verwendung eines Liners (100a; 100b; 200a; 200b; 300; 400) gemäß einem der Ansprüche 1 bis 12 zur Erstellung eines virtuellen 3D-Modells eines Körperteils (205a; 205b; 305) in einem Verfahren mit den folgenden Schritten:
- Formschlüssiges Anlegen des Liners (100a; 100b; 200a; 200b; 300; 400) am Körperteil (205a; 205b; 305);
- Akquirieren von dreidimensionalen Bilddaten des Körperteils (205a; 205b; 305) mittels eines Bildgebungsverfahrens;
- Segmentieren der Hautoberfläche in den dreidimensionalen Bilddaten anhand des verstärkten Kontrastes des Linermaterials bezüglich des Hautgewebes (201a; 201b; 301) des Körperteils (205a; 205b; 305); und
- Rekonstruieren eines 3D-Modells anhand der segmentierten 3D-Bilddaten, wobei das 3D-Modell die Oberflächenform des Körperteils (205a; 205b; 305) beschreibt.

15. Verwendung des Liners (100a; 100b; 200a; 200b; 300; 400) nach Anspruch 14, wobei das Verfahren auch ein Segmentieren der im Körperteil (205a; 205b; 305) enthaltenen Gewebearten (201a-204a; 201b-204b; 301-304) umfasst und das 3D-Modell die Gewebeverteilung in dem Körperteil (205a; 205b; 305) beschreibt.

## Claims

1. A liner (100a; 100b; 200a; 200b; 300; 400) for at least partially surrounding a body part (205a; 205b; 305) during an imaging process, which is suitable for simplifying the identification of the outer surface of the body part in the generated image data, wherein the liner (100a; 100b; 200a; 200b; 300; 400) is suitable for holding the body part (205a; 205b; 305) substantially in a given shape, and wherein the liner (100a; 100b; 200a; 200b; 300; 400) can be fitted in an approximately form-fit manner onto the skin surface of the body part, wherein the liner (100a; 100b; 200a; 200b; 300; 400) comprises a material which provides a base material and at least one contrast medium, wherein the contrast medium is distributed substantially homogenously within the base material, and wherein, during the use of the liner (100a; 100b; 200a; 200b; 300; 400) on the body part (205a; 205b; 305) in the imaging process, the contrast medium is suitable for intensifying the contrast of the liner material with reference to the skin tissue (201; 201; 301) in the generated image data relative to a contrast of a liner material comprising a base material with reference to the skin tissue (201a, 201b; 301).

2. The liner (100a; 100b; 200a; 200b; 300; 400) according to claim 1,
wherein the liner is suitable for holding the body part (205a; 205b; 305) in the shape of the body part (205a; 205b; 305) which is occupied when the body part (205a; 205b; 305) is disposed in a defined position or posture, especially in the case of a standing body.

3. The liner (100a; 100b; 200a; 200b; 300; 400) according to claim 1 or 2,
wherein the liner is suitable for completely surrounding a stump of an extremity, especially a stump of a thigh.

4. The liner (100a; 100b; 200a; 200b; 300; 400) according to any one of the preceding claims,
wherein the base material comprises polyurethane, silicon and/or thermoplastic elastomers.

5. The liner (100a; 100b; 200a; 200b; 300; 400) according to any one of the preceding claims, wherein the contrast medium is a contrast medium which acts in a contrast-intensifying manner in two or more different imaging processes.

6. The liner (100a; 100b; 200a; 200b; 300; 400) according to any one of claims 1 to 4,
wherein the contrast medium comprises iodine.

7. The liner (100a; 100b; 200a; 200b; 300; 400) according to claim 6,
wherein the mass ratio of the iodine to the base material is at least 1 x 10⁻⁵.

8. The liner (100a; 100b; 200a; 200b; 300; 400) according to any one of claims 1 to 4,
wherein the contrast medium comprises glycerol trinitrate.

9. The liner (100a; 100b; 200a; 200b; 300; 400) according to claim 8,
wherein the mass ratio of the glycerol trinitrate to the base material is at least 4 x 10⁻⁵.

10. The liner (100a; 100b; 200a; 200b; 300; 400) according to any one of the preceding claims,
wherein at least one further contrast medium, in each case for one further imaging process, is distributed homogenously in the base material of the liner (100a; 100b; 200a; 200b; 300; 400), wherein the contrast media do not substantially impair each other's contrast-intensifying action.

11. The liner (100a; 100b; 200a; 200b; 300; 400) according to any one of the preceding claims,
wherein the liner (100a; 100b; 200a; 200b; 300; 400) is embodied as a stocking open at one end or at both ends.

12. The liner (100a; 100b; 200a; 200b; 300; 400) according to any one of the preceding claims,
wherein the thickness of the liner material is at least 5 mm.

13. The use of a liner (100a; 100b; 200a; 200b; 300; 400) according to any one of claims 1 to 12 in a tensioning device for simulating the weight of a body part (205a; 205b; 305) acting on the body part (205a; 205b; 305) at different flexing angles (405) substantially parallel to the longitudinal axis of the body part (205a; 205b; 305) during an imaging process, wherein the tensioning device comprises a deflection roller (402) capable of being mounted in a height-adjustable manner, by means of which, via a weight (403) and a tensioning cord (404), a tensile force pre-definable in direction and magnitude can be exerted on the liner (100a; 100b; 200a; 200b; 300; 400) connected to the tensioning cord (404), so that, as a result, various flexing angles (405) of the body part (205a; 205b; 305) surrounded by the liner (100a; 100b; 200a; 200b; 300; 400) can be adjusted relative to the body.

14. The use of a liner (100a; 100b; 200a; 200b; 300; 400) according to any one of claims 1 to 12 for the preparation of a virtual 3-D model of a body part (205a; 205b; 305) in a process with the following steps:
- form-fit attachment of the liner (100a; 100b; 200a; 200b; 300; 400) to the body part (205a; 205b; 305);
- acquisition of three-dimensional image data of the body part (205a; 205b; 305) by means of an imaging process;
- segmentation of the skin surface in the three-dimensional image data on the basis of the intensified contrast of the liner material with reference to the skin tissue (201a; 201b; 301) of the body part (205a; 205b; 305); and
- reconstruction of a 3-D model on the basis of the segmented 3-D image data, wherein the 3-D model describes the surface shape of the body part (205a; 205b; 305).

15. The use of the liner (100a; 100b; 200a; 200b; 300; 400) according to claim 14, wherein the process also comprises a segmentation of the tissue types (201a-204a; 201b-204b; 301-304) contained in the body part (205a; 205b; 305), and the 3-D model describes the tissue distribution in the body part (205a; 205b; 305).

## Revendications

1. Manchon (100a ; 100b ; 200a ; 200b ; 300 ; 400) servant à entourer au moins en partie une partie corporelle (205a ; 205b ; 305) pendant un procédé d'imagerie, lequel est apte à simplifier l'identification de la surface extérieure de la partie corporelle dans les données d'image générées, le manchon (100a ; 100b ; 200a ; 200b ; 300 ; 400) étant apte à maintenir la partie corporelle (205a ; 205b ; 305) essentiellement dans une forme définie et le manchon (100a ; 100b ; 200a ; 200b ; 300 ; 400) pouvant être mis en place de manière approximative par complémentarité de forme contre la surface de la peau de la partie corporelle (205a ; 205b ; 305), le manchon (100a ; 100b ; 200a ; 200b ; 300 ; 400) étant constitué d'un matériau qui comprend un matériau de base et au moins un milieu contrastant, le milieu contrastant étant réparti essentiellement de manière homogène dans le matériau de base, et le milieu contrastant étant apte à amplifier par rapport au tissu cutané (201a ; 201b ; 301), lors de l'utilisation du manchon (100a ; 100b ; 200a ; 200b ; 300; 400) sur la partie corporelle (205a ; 205b ; 305) dans le procédé d'imagerie, le contraste du matériau du manchon par rapport au tissu cutané (201a ; 201b ; 301) dans les données d'image générées par rapport à un contraste d'un matériau de manchon constitué d'un matériau de base.

2. Manchon (100a ; 100b ; 200a ; 200b ; 300 ; 400) selon la revendication 1, le manchon étant apte à maintenir la partie corporelle (205a ; 205b ; 305) dans la forme de la partie corporelle (205a ; 205b ; 305) qui s'ajuste lorsque la partie corporelle (205a ; 205b ; 305) se trouve dans une position définie ou dans une posture définie, en particulier en position debout du corps.

3. Manchon (100a ; 100b ; 200a ; 200b ; 300 ; 400) selon la revendication 1 ou 2, le manchon étant apte à entourer intégralement un moignon des extrémités, en particulier un moignon de cuisse.

4. Manchon (100a ; 100b ; 200a ; 200b ; 300 ; 400) selon l'une quelconque des revendications précédentes, le matériau de base comprenant du polyuréthane, du silicone et/ou des élastomères thermoplastiques.

5. Manchon (100a ; 100b ; 200a ; 200b ; 300 ; 400) selon l'une quelconque des revendications précédentes, le milieu contrastant étant un milieu contrastant ayant un effet d'amplification de contraste dans deux procédés d'imagerie différents ou plus.

6. Manchon (100a ; 100b ; 200a ; 200b ; 300 ; 400) selon l'une quelconque des revendications 1 à 4, le milieu contrastant comprenant de l'iode.

7. Manchon (100a ; 100b ; 200a ; 200b ; 300 ; 400) selon la revendication 6, le rapport massique de l'iode par rapport au matériau de base étant d'au moins 1 x 10⁻⁵.

8. Manchon (100a ; 100b ; 200a ; 200b ; 300 ; 400) selon l'une quelconque des revendications 1 à 4, le milieu contrastant comprenant du trinitrate de glycérol.

9. Manchon (100a ; 100b ; 200a ; 200b ; 300 ; 400) selon la revendication 8, le rapport massique du trinitrate de glycérol par rapport au matériau de base étant au moins de 4 x 10⁻⁵.

10. Manchon (100a ; 100b ; 200a ; 200b ; 300 ; 400) selon l'une quelconque des revendications précédentes, au moins un autre milieu contrastant étant réparti de manière homogène dans le matériau de base du manchon (100a ; 100b ; 200a ; 200b ; 300 ; 400) pour chaque autre procédé d'imagerie respectif, les effets d'amplification respectifs des milieux contrastants n'étant essentiellement pas compromis.

11. Manchon (100a ; 100b ; 200a ; 200b ; 300 ; 400) selon l'une quelconque des revendications précédentes, le manchon (100a ; 100b ; 200a ; 200b ; 300 ; 400) étant réalisé sous la forme d'un bas ouvert d'un côté ou sur deux côtés.

12. Manchon (100a ; 100b ; 200a ; 200b ; 300 ; 400) selon l'une quelconque des revendications précédentes, l'épaisseur du matériau du manchon étant au moins de 5 mm.

13. Utilisation d'un manchon (100a ; 100b ; 200a ; 200b ; 300 ; 400) selon l'une quelconque des revendications 1 à 12 dans un dispositif de traction servant à simuler la force du poids de la partie corporelle (205a ; 205b ; 305) agissant au niveau d'une partie corporelle (205a ; 205b ; 305) selon différents angles de flexion (405) essentiellement de manière parallèle par rapport à l'axe longitudinal de la partie corporelle (205a ; 205b ; 305) au cours d'un procédé d'imagerie, le dispositif de traction comprenant un galet de renvoi (402) à agencer de manière réglable en hauteur, par l'intermédiaire duquel la force de traction pouvant être préalablement définie en termes de direction et d'intensité peut être exercée, au moyen d'un poids (403) et d'un câble de traction (404), sur le manchon (100a ; 100b ; 200a ; 200b ; 300 ; 400) relié au câble de traction (404) de sorte que de ce fait divers angles de flexion (405) de la partie corporelle (205a ; 205b ; 305) entourée par le manchon (100a ; 100b ; 200a ; 200b ; 300 ; 400) peuvent être ajustés par rapport au corps.

14. Utilisation d'un manchon (100a ; 100b ; 200a ; 200b ; 300 ; 400) selon l'une quelconque des revendications 1 à 12 servant à établir un modèle 3D virtuel d'une partie corporelle (205a ; 205b ; 305) dans un procédé comportant les étapes suivantes consistant à :
- mettre en place par complémentarité de forme le manchon (100a ; 100b ; 200a ; 200b ; 300 ; 400) sur la partie corporelle (205a ; 205b ; 305) ;
- acquérir des données d'image tridimensionnelles de la partie corporelle (205a ; 205b ; 305) au moyen d'un procédé d'imagerie ;
- segmenter la surface cutanée dans les données d'image tridimensionnelles à l'aide du contraste amplifié du matériau du manchon par rapport au tissu cutané (201a ; 201b ; 301) de la partie corporelle (205a ; 205b ; 305) ; et
- reconstruire un modèle 3D à l'aide des données d'image 3D segmentées, le modèle 3D décrivant la forme en surface de la partie corporelle (205a ; 205b ; 305).

15. Utilisation du manchon (100a ; 100b ; 200a ; 200b ; 300 ; 400) selon la revendication 14, le procédé comprenant également une segmentation des types de tissus (201a-204a ; 201b-204b ; 301-304) présents dans la partie corporelle (205a ; 205b ; 305) et le modèle 3D décrivant la répartition des tissus dans la partie corporelle (205a ; 205b ; 305).
